# EUROPEAN PATENT APPLICATION

(11) **EP 3 056 225 A1**
(43) Date of publication of application: **17.08.2016**
(21) Application number: 15000461.2
(22) Date of filing: 16.02.2015
(51) Int. Cl.: A61L 15/58, A61L 24/00

(54) **Debondable adhesive system**

(71) Applicant: Nitto Denko Corporation, Ibaraki-shi, Osaka 567-8680 (JP)
(72) Inventor: De Visscher, Geofrey, 1071 Chexbres (CH); Schüwer, Nicolas, 1007 Lausanne (CH)
(74) Representative: Hoffmann Eitle

(57) **Abstract**

The present invention relates to an adhesive system comprising a debondable adhesive layer and a hydrocolloid adhesive layer. The debondable adhesive layer can be in direct physical contact with the hydrocolloid adhesive layer, or a water permeable layer can be interposed between the debondable adhesive layer and the hydrocolloid adhesive layer to provide partial or complete physical separation of the two adhesive layers. The adhesive system is particularly suited for attaching and removing medical devices to and from the skin of a patient. The invention also relates to medical devises comprising the adhesive system.

## Description

### 1. FIELD OF THE INVENTION

The present invention relates to an adhesive system comprising a debondable adhesive layer and a hydrocolloid adhesive layer. The invention also relates to medical devices comprising the adhesive system. The adhesive system is particularly suited for attaching and removing medical devices, such as an ostomy faceplate, to and from the skin of a patient.

### 2. BACKGROUND OF THE INVENTION

Medical devices, such as the faceplate for an ostomy bag, are typically attached to a patient using an adhesive. The adhesive should be able to provide a strong and secure attachment of the medical device to the skin, thus avoiding unintentional detachment of the device, and in the case of ostomy bags, also leakage from the wearer's stoma. Once an attached medical device has served its purpose, its removal from the skin should ideally be painless and not cause any damage to the skin. This dichotomy of a secure attachment yet easy removal can be resolved by using a debondable adhesive.

Several technological solutions have been proposed. For instance, US 2010/0191204 A1 discloses a body waste collecting device comprising an adhesive composite that fastens a collecting bag to the skin. The adhesive composite is reported as being highly moisture resistant yet still capable of absorbing water. The adhesive composite comprises at least three layers, a backing layer, a moisture absorbing layer and a thin layer of vapour permeable, low-absorbing adhesive layer. US 4,701,169 describes an ostomy appliance comprising a body-attachable pad of skin-compatible adhesive material having a stomal orifice, and means whereby a plurality of ostomy bags can be attached in sequence to the pad by exposing a fresh region of adhesive each time it is desired to attach a clean ostomy bag. The appliance consists of numerous adhesive layers arranged so that the peeling off of a first layer exposes a second layer, the latter not being accessible to the user until the first layer has been peeled off. The adhesives are composed of water swellable hydrocolloids. GB 2322302 A teaches the use of an ostomy pouch coupling for connecting an ostomy pouch to the stoma of a patient. The device comprises a plurality of apertured sheets of paper or plastics film adhered in face-to-face contact by respective layers of a differential adhesive. A layer of hydro-colloid adhesive is disposed on the outer surface of the substrate for semi-permanently adhering the coupling to the skin.

A debondable adhesive is an adhesive that can be switched from a comparatively high-tack state to a comparatively low-tack state by the application of a trigger such as cooling (EP 0 471 767 A1; US 5,156,911), UV or visible light (EP 2 371 920 A1), electric current (WO 2001/005584 A1) or the addition of a detackifying agent (US 4,324,595; US 6,436,227; WO 2003/061720 A1). However, a problem with current debondable adhesives is that fluid originating from the body (e.g. sweat, excrement, etc.) and moisture (e.g. from bathing, sports, etc.), or a combination thereof, can compromise the adhesiveness of the system and also the integrity of the seal against the body. In the case of ostomy bags, this results in users being concerned with the embarrassment and inconvenience of the ostomy appliance leaking or becoming detached, not to mention skin problems that occur when excrement is kept in contact with the skin. Furthermore, the form and size of certain medical devices, particularly ostomy bags, hinders the application of a trigger to the adhesive such that the benefit of using a debondable adhesive is limited.

The present invention overcomes the aforementioned problems by ensuring that moisture and water are drawn away from the debondable adhesive that is in contact with the user's skin, and by allowing the optional removal of the medical device before a trigger is applied to the debondable adhesive.

### 3. SUMMARY OF THE INVENTION

The present invention provides an adhesive system for a medical device. The adhesive system comprises a debondable adhesive layer and a hydrocolloid adhesive layer. The debondable adhesive layer of the adhesive system attaches to the patient's skin. The hydrocolloid adhesive layer is removed from the patient's skin and draws moisture away from the debondable adhesive layer. The hydrocolloid adhesive layer can be in direct physical contact with the debondable adhesive layer, or it may be separated (wholly or partially) therefrom by another layer interposed between the two adhesive layers. For example, a water permeable layer may be interposed between the hydrocolloid adhesive layer and the debondable adhesive layer. The interposed layer may be situated such that no direct physical contact is made between the surfaces of the two adhesive layers. In preferred embodiments the peel force of the hydrocolloid and debondable adhesive layers are such that the hydrocolloid adhesive layer can be peeled away (with or without any interposed water permeable layer) so as to leave the debondable adhesive layer attached to the patient. The debondable adhesive layer can then be treated with a debonding trigger to reduce its peel force thereby allowing it to be easily removed from the skin of a patient. The adhesive system is particularly suited for attaching medical devices such as wound dressings, prostheses, ostomy faceplates or bags, electrodes, diagnostic devices, insulin pumps and catheters. The invention is also directed to medical devices comprising the adhesive system of the invention.

### 4. BRIEF DESCRIPTION OF THE DRAWINGS

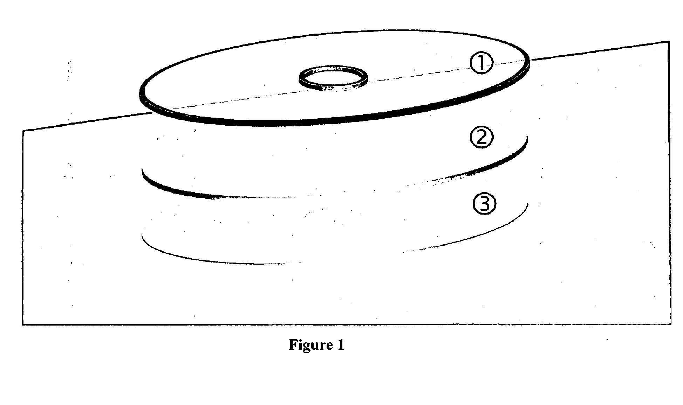

Figure 1 depicts one embodiment of the invention. In this figure, an adhesive system (exploded view) comprising a debondable adhesive layer (3) and a hydrocolloid adhesive layer (2) is used to attach a medical device (1), in this embodiment an ostomy faceplate, to the skin of a patient (shaded lower area of the figure).

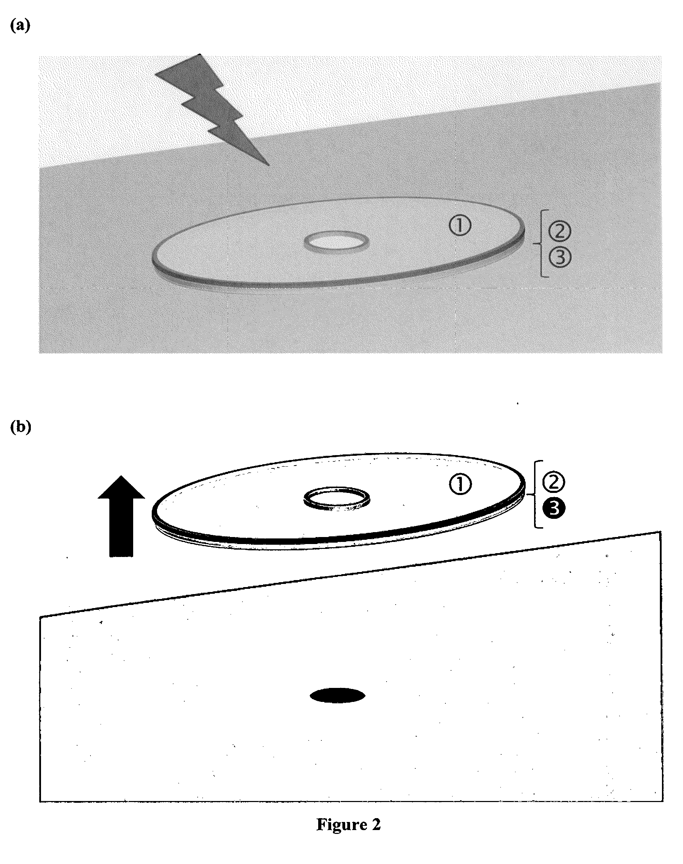

Figure 2 depicts one way to remove a medical device that has been attached to the skin of a patient around a stoma by the adhesive system shown in Figure 1. Upon application of a trigger (e.g. light, as depicted by the jagged arrow), the peel force of the debondable adhesive layer (3) can be reduced (Figure 2a). The medical device (1) can then be removed together with the hydrocolloid adhesive layer (2) and the now-debonded layer (3) as indicated by the blacked-out reference numeral (Figure 2b).

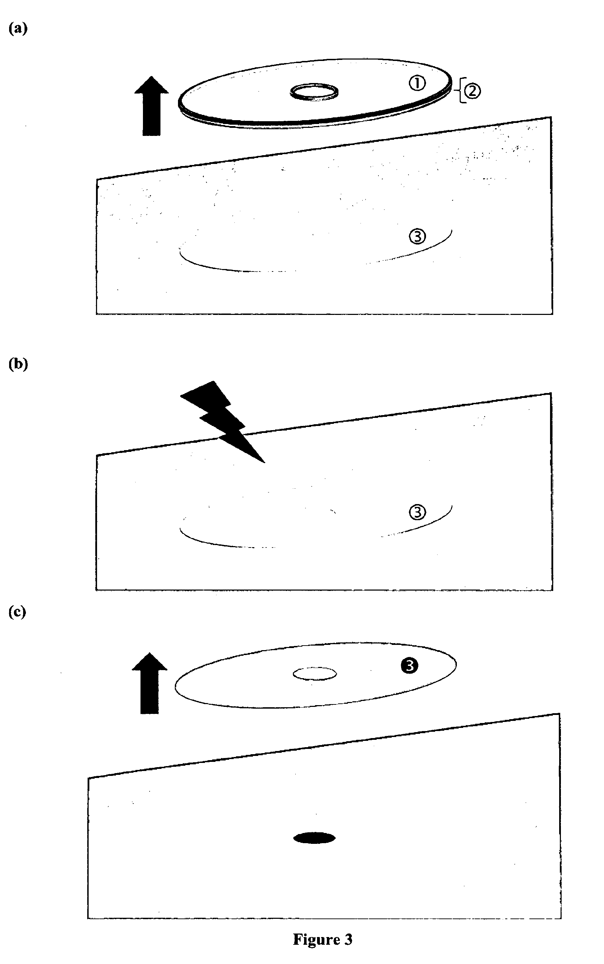

Figure 3 depicts another way to remove a medical device that has been attached to the skin of a patient by the adhesive system shown in Figure 1. In this case the respective peel forces of the hydrocolloid adhesive layer (2) and the debondable adhesive layer (3) are such that the medical device (1) and the hydrocolloid adhesive layer (2) can be removed while leaving the debondable adhesive layer (3) still attached to the patient's skin (Figure 3a). A trigger can then be applied to the debondable adhesive layer (3) to reduce its peel force (Figure 3b) thereby allowing it to be removed easily from the skin of the patient (Figure 3c).

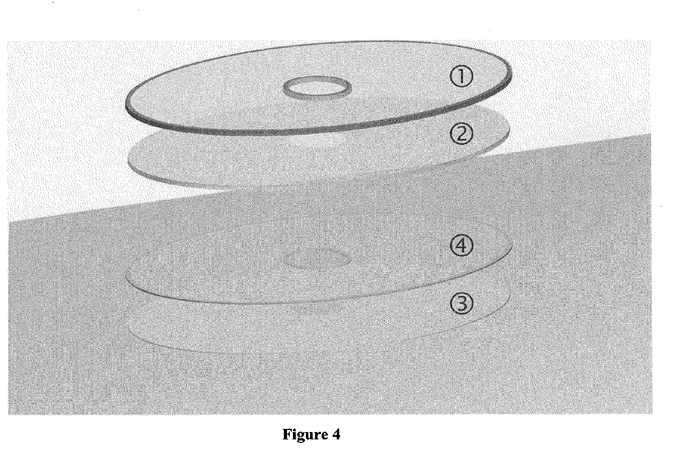

Figure 4 depicts a further embodiment of the invention. In this figure, an adhesive system comprising a debondable adhesive layer (3) and a hydrocolloid adhesive layer (2), with a water permeable layer (4) interposed between the two adhesive layers, is used to attach a medical device (1), in this embodiment an ostomy faceplate, to the skin of a patient.

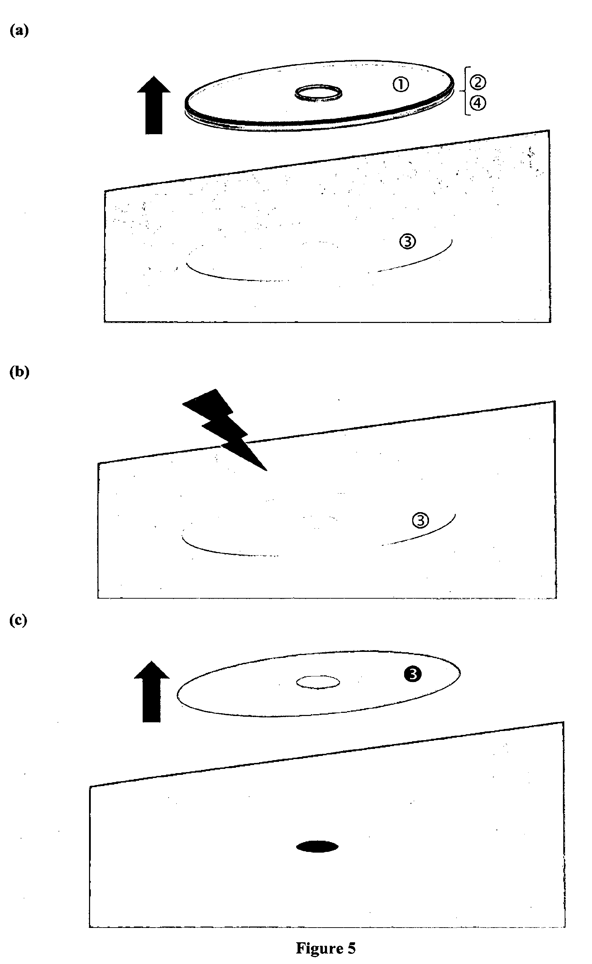

Figure 5 depicts one way to remove a medical device that has been attached to the skin of a patient by the adhesive system shown in Figure 4. In this case the respective peel forces of the hydrocolloid adhesive layer (2) and the debondable adhesive layer (3) are such that the medical device (1), the hydrocolloid adhesive layer (2), and the water permeable layer (4) can be removed while leaving the debondable adhesive layer (3) still attached to the patient's skin (Figure 5a). A trigger can then be applied to the debondable adhesive layer (3) to reduce its peel force (Figure 5b) thereby allowing it to be removed easily from the skin of the patient (Figure 5c).

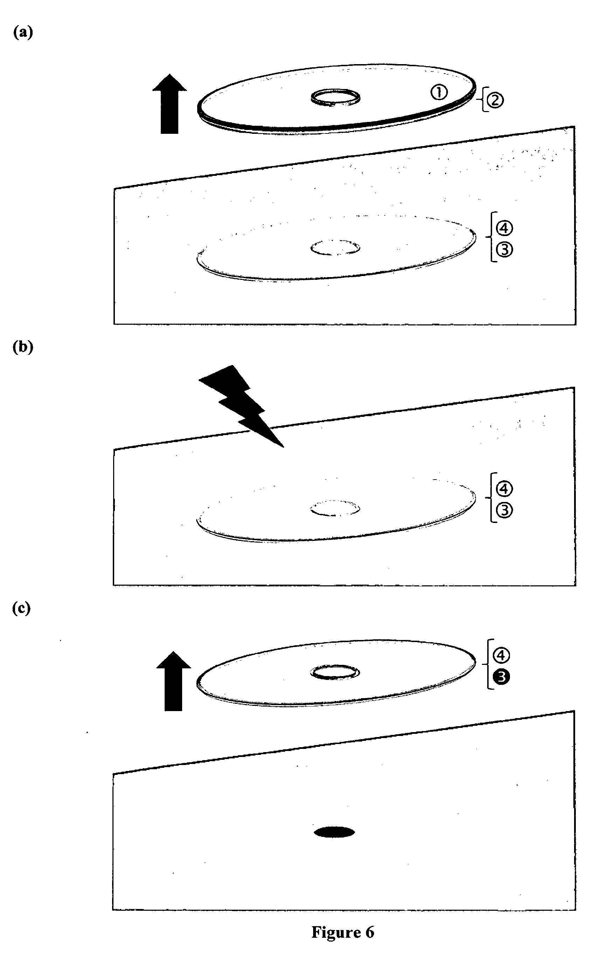

Figure 6 depicts another way to remove a medical device that has been attached to the skin of a patient by the adhesive system shown in Figure 4. In this case the respective peel forces of the hydrocolloid adhesive layer (2) and the debondable adhesive layer (3) are such that the medical device (1) and the hydrocolloid adhesive layer (2) can be removed while leaving the water permeable layer (4) and the debondable adhesive layer (3) still attached to the patient's skin (Figure 6a). A trigger can then be applied to the debondable adhesive layer (3) to reduce its peel force (Figure 6b) thereby allowing it and the water permeable layer (4) to be removed easily from the skin of the patient (Figure 6c).

### 5. DETAILED DESCRIPTION OF THE INVENTION

As used herein, the terms "comprises", "comprising", "includes", "including", "has", "having" or any other variation thereof, are intended to cover a non-exclusive inclusion. For example, an adhesive system that comprises a list of components is not necessarily limited to only those components but may include other components that are not expressly listed or inherent to such a composition. That said, the terms "comprises", "comprising", "includes", "including", "has", "having" or any other variation thereof also cover the disclosed embodiment having no further additional components (i.e. consisting of those components). By way of example, an adhesive system comprising a debondable adhesive layer and a hydrocolloid adhesive layer discloses the adhesive system with just these components as well as an adhesive system comprising these components along with other unmentioned components (e.g. a water permeable layer, ostomy faceplate and/or a release liner).

Also, the indefinite articles "a" and "an" preceding an element or component of the invention are intended to be non-restrictive regarding the number of instances (i.e. occurrences) of the element or component. Therefore "a" or "an" should be read to include one or at least one, and the singular word form of the element or component also includes the plural unless the number is obviously meant to be singular. By way of example, reference to a hydrocolloid adhesive layer comprising a polymer should be understood to mean that the hydrocolloid adhesive layer comprises one or at least one type of that polymer unless specified otherwise.

Further, when an aspect of the invention is described as being 'preferred', it should be understood that this preferred aspect of the invention can be combined with other preferred aspects of the invention.

### 5.1 Debondable adhesive layer

*"Debondable adhesive",* or switchable adhesive as it is sometimes known, is a term that is understood in the art. Any debondable adhesive that is suitable for application to the human skin can be used for the adhesive system of the present invention. Preferably, the debondable adhesive is one whose peel force on the ear of a pig as measured by Test Method A of ASTM D3330M-04(2010) can be reduced on demand by 60% or more within 15 minutes of a suitable trigger being applied to the adhesive. *"On demand"* means that the peel force can be reduced controllably by the application of a trigger (e.g. by heating or cooling (collectively referred to as 'temperature' triggered unless indicated otherwise), exposure to electromagnetic radiation (such as UV or visible light), exposure to chemical stimulation, electrical stimulation, mechanical stimulation, exposure to ultrasonic, sonic or infrasonic waves, or exposure to any other detackifying agent).

Any debondable adhesive that is suitable for application to the human skin can be used for the adhesive system of the present invention. Suitable debondable adhesives include those described in the prior art documents that are mentioned above under the heading "Background of the invention". Other debondable adhesives include the UV debondable adhesives that are described in US 4,286,047, US 4,968,559, US 5,118,567 , US 5,187,007 and JP 3043988; visible light debondable adhesives that are described in EP 0 863 775, US 6,184,264 and US 6,610,762; and heat debondable adhesives that are described in WO 2013/012973 A1, WO2000/040648 A1, and US 8,409,703.

For instance, temperature debondable adhesives containing a crystalline polymeric additive as described in US 5,412,035 can be used. The crystalline polymeric additive has a weight-average molecular weight of less than 25,000 and a melting point greater than 23°C. The crystalline polymeric additive may be a side chain crystallizable polymer, including but not limited to, polymers of one or more monomers such as substituted or unsubstituted acrylates, fluoroacrylates, vinyl esters, acrylamides, maleimides, α-olefins, p-alkyl styrenes, alkylvinyl ethers, alkylethylene oxides, triglycerides, alkyl phosphazenes and amino acids; polyisocyanates, polyurethanes, and polysiloxanes. Suitable base resins of the debondable adhesive include, but are not limited to polyacrylate or styrene/butadiene copolymers.

The temperature debondable adhesive may also be an adhesive comprising an elastomer and a crystallizable abietic acid derivative as tackifier, as described in US 7,399,800. The abietic acid derivatives are derivatives of abietic acid, or related resin acids, including but not limited to, neoabietic acid, palustric acid, dehydroabietic acid, pimaric acid, and isopimaric acid. Suitable crystallizable abietic acid derivatives contain a crystallizable group, including but not limited to, a linear or branched, substituted or unsubstituted alkyl or trans alkenyl group having from 12 to 30 carbon atoms, a fluoroalkyl or trans fluoroalkenyl group having from 12 to 30 carbon atoms, phenyl, benzyl, phenolic, naphthalenic, or hydroquinoid. In one embodiment, the crystallizable abietic acid derivative is hexadecyl ester of abietic acid (i.e., cetyl abietate). Suitable elastomers include, but are not limited to, thermoplastic rubbers, natural rubbers, butyl rubbers, polyisobutylene polymers, vinyl ether polymers, ethylene/acrylic copolymers, and silicone-based rubbers.

The temperature debondable adhesive may also be an adhesive comprising an elastomer and a crystallizable pinene-based tackifier, as described in US 2006/0235149. The crystallizable pinene-based tackifiers are copolymers comprising at least one pinene monomer including, but not limited to, *β*-pinene and *α*-pinene oxide, and at least one co-monomer that is capable of cationically polymerizing with the pinene monomer and has at least one crystallizable group. A mixture of pinene monomers and co-monomers may also be used. Suitable comonomers include vinyl ethers, alkoxy styrenes, cyclopentadienes, and dicyclopentadienes having at least one crystallizable group. Exemplary crystallizable groups include, but are not limited to, linear or branched, substituted or unsubstituted alkyl or trans- alkenyl groups having from 12 to 30 carbon atoms, fluoroalkyl or trans-fluoroalkenyl groups having from 12 to 30 carbon atoms, phenyl, benzyl and naphthalenyl. Suitable elastomers include, but are not limited to, thermoplastic rubbers, natural rubbers, butyl rubbers, polyisobutylene polymers, vinyl ether polymers, ethylene/acrylic copolymers, and silicone-based rubbers.

Additionally, the temperature debondable pressure sensitive adhesive may comprise at least one pressure sensitive adhesive which does not exhibit temperature debondable properties and at least one crystallizable oil, as described in US 12/771,078. Suitable non-debondable pressure sensitive adhesives are described below under the description of the second adhesive layer. Suitable crystallizable oils are oils that have a crystalline melting point (CMP) that occurs at a temperature within the temperature range of the rubbery plateau region of the pressure sensitive adhesive being used, specifically between the glass transition temperature (T1) and the temperature at which the material flows as a low viscosity liquid (T2). Therefore, the choice of a suitable crystallizable oil depends on the pressure sensitive adhesive of interest. The temperature range of the rubbery plateau region of the pressure sensitive adhesive may be found in the literature or may be determined experimentally using methods known in the art (e.g., thermomechanical analysis). Then, oils that have a crystalline melting point within the rubbery plateau region of the pressure sensitive adhesive of interest may be found in the literature (e.g., The Handbook of Chemistry and Physics, CRC Press, Boca Raton, Fla.). The debonding temperature of the adhesive may be controlled by selecting crystallizable oils having different crystalline melting temperatures within the rubbery plateau. In general, a crystallizable oil having a crystalline melting point close to the desired debonding temperature is used. Suitable crystallizable oils are branched or unbranched oils that have a crystalline melting point, and include, but are not limited to, hexadecane (CMP about 18° C.), 1-hexadecanol (CMP about 48-50° C.), 1-tetradecane (CMP about 6° C.), 1-tetradecanol (CMP about 35-39° C.), octadecane (CMP about 26-29° C.), and octadecanol (CMP of about 56-59° C.). These oils are particularly useful in combination with polystyrene-block-polyisoprene-block-polystyrene (rubbery plateau from T1 about -70° C. to T2 about 95° C.) based pressure sensitive adhesives. The crystallizable oil is used at a concentration of at least about 20%, more particularly, at least about 25% by weight relative to the total weight of the adhesive composition. In one embodiment, the first adhesive layer comprises a styrene-isoprene-styrene triblock copolymer which has a styrene content of about 14% to about 22% by weight, abietic acid as tackifier, and 1-hexadecanol as crystallizable oil. In another embodiment, the first adhesive layer comprises a styrene-isoprene-styrene triblock copolymer which has a styrene content of about 14% to about 22% by weight, poly-*β*-pinene as tackifier, and 1-hexadecanol as crystallizable oil.

All of the aforementioned temperature debondable pressure sensitive adhesives lose adhesive strength when heated to a temperature above the debonding temperature. Additionally, a temperature debondable pressure sensitive adhesive that loses adhesive strength upon cooling, such as those described in US 5,156,911 and US 5,387,450 may be used. Those temperature debondable pressure sensitive adhesives comprise a side-chain crystallizable polymer having a heat of fusion greater than 20 Joules/g and a melting point or first order transition temperature in the range of about 20° C. to 35° C. The backbone of the polymer may be any organic structure (aliphatic or aromatic hydrocarbon, ester, ether, amide, etc.) or an inorganic structure (sulfide, phosphazine, silicone, etc.), and may include spacer linkages which can be any suitable organic or inorganic unit, for example ester, amide, hydrocarbon, phenyl, ether, or ionic salt (e.g., a carboxyl-alkyl ammonium or sulphonium or phosphonium ion pair or other known ionic salt pair). The side-chain may be aliphatic or aromatic or a combination of aliphatic and aromatic, and is capable of entering into a crystalline state. Common examples are: linear aliphatic side-chains of at least 10 carbon atoms, e.g., C14-C22 acrylates or methacrylates, acrylamides or methacrylamides, vinyl ethers or esters, siloxanes or alpha olefins; fluorinated aliphatic side-chains of at least 6 carbons; and p-alkyl styrene side-chains wherein the alkyl is of 8 to 24 carbon atoms.

As noted above, there is no particular limitation regarding the adhesive systems that can be used for making the adhesive compositions of the present invention. Adhesive systems may in particular be pressure-sensitive adhesive systems. Typical representatives of such systems, which are all useful for the present invention, are acrylics, rubbers, silicones, polyurethanes, polyesters, polyethers and EVA systems. For instance, any of the pressure-sensitive adhesive systems described in "Adhesion Science and Engineering" edited by M. Chaudhury and A.V. Pocius, Elsevier B.V., 2002; "Pressure-sensitive Adhesives and Applications" by I. Benedek, 2nd Ed., Marcel Dekker Inc., 2004; "Technology of Pressure-Sensitive Adhesives and Products" edited by I. Benedek and M.M. Feldstein, CRC Press, 2009; as well as Zbigniew Czech and Agnieszka Kowalczyk (2011) "Pressure-Sensitive Adhesives for Medical Applications", Wide Spectra of Quality Control, Dr. Isin Akyar (Ed.), ISBN: 978-953-307-683-6, InTech, (available from: http://www.intechopen.com/books/wide-spectra-of-quality-control/pressure-sensitive-adhesives-for-medicalapplications) can be used in the present invention.

The adhesive may be any adhesive which includes a component that allows the adhesive to debond upon the application of electromagnetic radiation such as UV or visible light. For instance, reference is made to EP Application Nos. 14 173 246.1 and 14 173 248.7 for suitable adhesive compositions for use in the present invention.

The debondable adhesive composition of the present invention can be a composition as disclosed in EP application 14173246.1. For example, the debondable adhesive composition may comprise an adhesive system and a debonding component, wherein the debonding component is an organic compound having a molecular weight of from 200 g/mol to 10000 g/mol, which debonding component contains two or more photodimerizable groups. Preferably, the debonding component is a compound characterized by the following general formula (I):

COR(-LIN-PDM)ₙ (I)

wherein COR represents a core moiety selected from hydrocarbon groups having from 2 to 20 carbon atoms and n valencies;
each LIN group represents a linker moiety independently selected from a covalent bond, C1-6 alkylene, C2-6 alkenylene, C2-6 alkynylene, -O-C(=O)-, -C(=O)-, -O-, -C(=O)-O-, -N(R¹)-C(=O)-, -N(R¹)-, -C(=O)-N(R¹)-, -O-C(=O)-O-, -O-C(=O)-N(R¹)-, -N(R¹)-C(=O)-O-, -O-C(=N(R¹))-, -C(=N(R¹))-O-, -O-C(=N(R¹))-N(R¹)-, - N(R^{l})-C(=N(R¹))-O-, -N(R¹)-C(=N(R¹))-N(R¹)-, and combinations of two or more of these groups, wherein R¹ may be selected from hydrogen, C1-6 alkyl, C2-6 alkenyl, C2-6 alkynyl, C6-10 aryl, 5 to 10 membered saturated, unsaturated or aromatic heterocycle containing 1 to 4 heteroatoms selected from O, N and S, wherein each of these groups may itself optionally carry one to three substituents independently selected from halogen, C1-6 alkyl, C1-6 alkoxy, -NH₂ and - NO₂;
each PDM group represents a photodimerizable group; and
n represents an integer of 2 to 10;
such that the molecular weight of the debonding component of formula (I) is in the range of from 200 g/mol to 3000 g/mol, preferably in the range of from 300 to 1500 g/mol.

Alternatively, LIN in Formula (I) may be absent. The absence of LIN is an advantageous option especially if COR and PDM contain functional groups that can be coupled with each other, such as a carboxylic acid group and a hydroxyl or amino group and if the direct coupling of two or more PDM groups to COR is sterically possible.

The debondable adhesive composition of the present invention can be a composition comprising an adhesive system and a debonding component wherein the debonding component is an oligomeric compound which contains two or more repeating units of general formula (II):

-(REP (-LIN-PDM))- (II)

wherein REP represents a repeating unit core moiety derived from a monomeric compound selected from amino acids, saccharides, and vinylalcohol;
each LIN group represents a linker moiety independently selected from a covalent bond, C1-6 alkylene, C2-6 alkenylene, C2-6 alkynylene, -O-C(=O)-, -C(=O)-, -O-, -C(=O)-O-, -N(R¹)-C(=O)-, -N(R¹)-, -C(=O)-N(R¹)-, -O-C(=O)-O-, -O-C(=O)-N(R¹)-, -N(R¹)-C(=O)-O-, -O-C(=N(R¹))-, -C(=N(R¹))-O-, -O-C(=N(R¹))-N(R¹)-, - N(R¹)-C(=N(R¹))-O-, -N(R¹)-C(=N(R¹))-N(R¹)-, and combinations of two or more of these groups, wherein R¹ may be selected from hydrogen, C1-6 alkyl, C2-6 alkenyl, C2-6 alkynyl, C6-10 aryl, 5 to 10 membered saturated, unsaturated or aromatic heterocycle containing 1 to 4 heteroatoms selected from O, N and S, wherein each of these groups may itself optionally carry one to three substituents independently selected from halogen, C1-6 alkyl, C1-6 alkoxy, -NH₂ and - NO₂;
each PDM group represents a photodimerizable group; and
wherein the molecular weight of the debonding component of general formula (II) is in the range of from 500 g/mol to 6000 g/mol, preferably in the range of from 600 to 4000

In formula (I) and (II) above, COR is preferably derived from a tri-, tetra- or hexafunctional alcohol or amine compound and wherein LIN is optionally absent; and/or the PDM group is preferably a group derived from a molecule selected from coumarin, stilbene, anthracene, cinnamic acid, thymine and substituted forms thereof by abstracting one of its hydrogen atoms to provide for the free valency for covalent bonding to the LIN group.

The debondable adhesive composition of the present invention can be a composition as disclosed in EP application 14173248.7. For example, the debondable adhesive composition of the present invention can be a composition comprising:
(A) an ethylenically unsaturated polycondensate of
   a) a dicarboxylic acid, and
   b) a diol, and
   c) an ethylenically unsaturated dicarboxylic acid and/or an ethylenically unsaturated diester monomer.
      and
(AB) a cross-linking agent having at least 3 functional groups per molecule;
   wherein the unsaturated polycondensate and the cross-linking agent are capable of reacting with each other to form an adhesive substance via a chemical reaction that does not involve the ethylenically unsaturated bonds of the repeating units contained in the polycondensate (A).

Preferably, the the dicarboxylic acid (a), the diol (b), and/or the ethylenically unsaturated dicarboxylic acid monomer, and/or an ethylenically unsaturated diester monomer (c) are selected from general formula (III) to (V), respectively:

R^{a}OOC-R¹-R²-R³-COOR^{b} (III)

wherein R¹ is absent or selected from C1-15 alkylene, C11-15 alkenylene, and C2-15 alkynylene, wherein each of these groups is optionally substituted; R² is selected from C1-12 alkylene, C11-12 alkenylene, and C2-12 alkynylene, each of which may contain one or two 5-7-membered rings, which may optionally be condensed wherein each of these groups is optionally substituted; R³ is absent or selected from C1-15 alkylene, C11-15 alkenylene, and C2-15 alkynylene wherein each of these groups is optionally substituted; each of R^{a} and R^{b} is independently selected from hydrogen, alkyl groups having 1 to 24 carbon atoms, and glyceryl, wherein the dicarboxylic acid (a) is preferably a dimer of fatty acids;

HO-R⁴-R⁵-R⁶-OH (IV)

wherein R⁴ is absent or selected from C1-15 alkylene, C2-15 alkenylene, and C2-15 alkynylene, wherein each of these groups is optionally substituted; R⁵ is selected from C1-12 alkylene, C2-12 alkenylene, and C2-12 alkynylene, each of which may contain one or two 5-7-membered rings, which may optionally be condensed, wherein each of these groups is optionally substituted; R⁶ is absent or selected from C1-15 alkylene, C2-15 alkenylene, and C2-15 alkynylene, wherein each of these groups is optionally substituted, wherein the diol (b) is preferably a dimer of fatty alcohols; and

R^{c}OOC-R⁷-COOR^{d} (V)

wherein R⁷ is selected from C1-10 alkylene, C2-10 alkenylene, and C3-10 alkynylene and wherein each of these groups may be unsubstituted or substituted by 1-3 substituents, wherein said substituents are preferably selected from C1-8 alkyl, C1-8 alkenyl, C2-8 alkynyl, C1-6 alkoxy, and -R⁸-COOR⁹ wherein R⁷ and/or at least one of its substituents is selected such that one or more carbon-carbon double bonds is/are present in the part of the molecule formed by R⁷ and its substituents, wherein such carbon-carbon double bond(s) may also be formed between a carbon atom of R⁷ and the carbon atom of the substituent, which is covalently bonded to said carbon atom of R⁷, wherein R⁸ is C1-3 alkylene, C2-3 alkenylene or C2-3 alkynylene and R⁹ is C1-6 alkyl, wherein each of R^{c} and R^{d} is independently selected from hydrogen and alkyl groups having 1 to 6 carbon atoms and wherein the one or more carbon-carbon double bonds may each independently have cis or trans (Z or E) stereochemistry.

In an even more preferred embodiment, dicarboxylic acid (a) is selected from itaconic acid and/or citraconic acid and/or mesaconic acid and/or aconitic acid, and/or their respective dialkyl esters.

Any of the above compositions may further comprise an oil additive, preferably an oil additive selected from sorbitan mono- or di-esters of fatty acids, polyethylene glycol diester, sesame oil, isosorbide diester, mixtures of isosorbide diesters, sorbitol mono- and diesters, oleate esters, isopropyl myristate or mixtures thereof.

The debondable adhesive layer may have any thickness that is suitable for its intended use. For instance, a comparatively light medical device requires a thinner debondable adhesive layer than a comparatively heavier medical device. By way of example, when the debondable adhesive layer is used to attach an ostomy plate, the preferred thicknesses for the debondable adhesive layer is 10 micron or more, more preferably 20 micron or more, more preferably 30 micron or more, more preferably 50 micron or more, more preferably 70 micron or more and more preferably 100 micron or more in view of providing sufficient adhesion for the ostomy plate and ostomy bag as it fills with bodily waste. In terms of providing a more conformable and comfortable layer, the debondable adhesive layer preferably has a thickness of 1000 micron or less, more preferably 800 micron or less, more preferably 600 micron or less, more preferably 500 micron or less, more preferably 400 micron or less, and most preferably 300 micron or less. Any of the preferred lower limits for the debondable adhesive thickness can be combined with any of the preferred upper limits to define further suitable thickness ranges for the present invention. As an example, further exemplary ranges for the debondable adhesive thickness include: 10-1000 micron, 20-800 micron, 30-600 micron, 50-500 micron, 70-400 micron, 100-300 micron, 50-1000 micron, 100-1000 micron and 10-300 micron. The above values represent the thickness of the debondable adhesive layer before the adhesive system is used to attach a device to the skin. Where the thickness of the debondable adhesive layer varies across the adhesive system, then the above values refer to the average thickness of the debondable adhesive layer. The thickness of the debondable adhesive layer may be adjusted according to need and the debonding mechanism employed. For example, when light-triggered debonding is employed then the debondable adhesive layer can be made comparatively thinner than a debondable adhesive layer where a temperature-triggered debonding mechanism is employed.

The debondable adhesive layer is preferably in the form of a continuous sheet, but may also take the form of a sheet interrupted by punched holes that influence moisture vapour transmission rate or any other gaps required to attach a medical device to the skin. For instance, the debondable adhesive layer may be in the form of a sheet with a central aperture (e.g. to allow access to the stoma of a patient). The debondable adhesive layer may be patterned or may include a patterned shape (e.g. straight lines, curved lines, chevrons, island pattern, etc.) to increase or decrease adhesive strength and/or shear adhesive strength as required.

### 5.2 Hydrocolloid adhesive layer

In addition to the debondable adhesive layer, the adhesive system of the present invention comprises a hydrocolloid adhesive layer. The hydrocolloid adhesive layer readily absorbs moisture and by doing so effectively transports moisture away from the debondable adhesive layer where moisture, if retained, might interfere with the adhesive properties of the debondable adhesive layer.

A major problem with many conventional hydrocolloid adhesive compositions is their susceptibility to breakdown upon exposure to wound exudate and body fluids (i.e. their lack of structural integrity after being hydrated). When the compositions are used as skin barriers, e.g., around stomas, some absorption of fluid is desirable, but excessive swelling causes the composition to lose its moisture seal with the skin. Leakage occurs and the barrier must be replaced more often than is desirable. Furthermore, when the adhesive compositions are used as wound dressings, the compositions tend to dissolve upon exposure to wound exudate and form a gel on the surface of the wound. When the dressing is removed, a residue remains on the wound requiring removal, typically by irrigation. When this breakdown occurs the dressings may also lift off the wound and allow leakage of wound exudates and/or body effluent onto clothing and bedding. Hydrocolloid adhesives have also been known to cause itching and local irritation to the skin. In the present invention these problems are avoided by situating the hydrocolloid adhesive layer away from the skin. Instead, the debondable adhesive layer attaches to the skin so as to provide the skin barrier thus reducing the risk that leakage will occur when the hydrocolloid adhesive layer swells, reduce the risk that wounds will become contaminated by hydrogel material, or reduce skin irritation such as itchiness.

Furthermore, by including a hydrocolloid adhesive layer in the adhesive system, the relative peel forces of the adhesive layers in the system can be tailored such that the hydrocolloid layer peels away from the patient more readily than the debondable adhesive. By first removing the hydrocolloid layer, greater access to the debondable adhesive layer can be gained when it comes time to apply a debonding trigger.

Any hydrocolloid-containing adhesive can be used in the present invention. Typically, these compositions comprise a blend of a polymer matrix, such as a rubbery elastomer like polyisobutylene, in combination with one or more water-soluble or water-swellable hydrocolloids, such as a dry powdered mixture of pectin, gelatin and carboxymethylcellulose. Suitable hydrocolloids for the adhesive system of the present invention include synthetic polymers prepared from single or multiple monomers, naturally occurring hydrophilic polymers or chemically modified naturally occurring hydrophilic polymers. The hydrocolloid polymers may be linear or cross-linked. Natural or chemically modified natural polymers include, but are not limited to, cellulosics, such as carboxymethyl cellulose (CMC), chitosan, pectin, guar gum, starches, dextrines, collagens or gelatine. Synthetic polymers include, but are not limited to, polyacrylic acid, polyvinylealcohol/acetate, polyhydroxyalkyl acrylates and methacrylates, polyacrylamides, polystyrene sulfonates, polyvinylpyrrolidone, polyglycols, or copolymers, grafts or mixtures of these. In one embodiment of the invention, the hydrocolloid adhesive layer comprises hydrocolloid particles selected from cellulosics, chitosan, pectin, guar gum, starches, dextrines, collagens, gelatin, polyacrylic acid, polyvinylealcohol/acetate, polyhydroxyalkyl acrylates or methacrylates, polyacrylamides, polystyrene sulfonates, polyvinylpyrrolidone, polyglycols, and copolymers, grafts or mixtures of these. In a more preferred embodiment of the invention, the hydrocolloid adhesive layer comprises hydrocolloid particles selected from cellulosics.

Suitable hydrocolloid adhesive layers that can be used in the present invention are described in US 3,980,084, US 3,877,431, US 3,339,546, US 4,231,369, US 4,367,732, US 4,867,748, US 5,714,225 and EP 0 343 807. A commercially available example of a composition of this type is STOMAHESIVE™ (ConvaTec).

Preferably, the hydrocolloid adhesive layer comprises 5 to 70 wt.% of hydrocolloid based on the total weight of the hydrocolloid adhesive. The total amount of hydrocolloid is more preferably 10-55% by weight of the hydrocolloid adhesive, even more preferably 15-50% by weight, and most preferably from 20 to 40% by weight of the total composition. Any of the disclosed wt.% lower limits for the total amount of hydrocolloid can be combined with any of the disclosed wt.% upper limits to define further suitable wt.% ranges for the purposes of this invention. For instance, further exemplary ranges for the amount of hydrocolloid in the hydrocolloid adhesive layer include 5 to 55 wt.%, 10 to 50 wt.% and 15 to 70 wt.%.

The hydrocolloid may be in the form of particles having a size (D50) in the range of from 10 to 300 µm. Preferred upper limits for this range are 250, 200 and 150 µm. By D50 is meant that 50 v/v % of the hydrocolloid particles are within the stated size range. Major improvements in moisture transmission from the debondable adhesive to the hydrocolloid adhesive are obtained by the addition of small size hydrocolloid particles. This makes it possible to prepare comparatively thinner layers of adhesive. In this respect, the D50 size of the hydrocolloid particles is preferably below 50 µm, more preferably below 40 µm and most preferably below 30 µm.

The hydrocolloids in the adhesive layers may be a single type of hydrocolloid or a mixture of hydrocolloids. The hydrocolloid adhesive layer may comprise two or more layers of hydrocolloid adhesive. For instance, the hydrocolloid adhesive layer may comprise a first and second layer of hydrocolloid adhesive, wherein the first hydrocolloid adhesive layer (facing the water permeable layer) comprises a hydrocolloid providing a higher moisture absorption capacity and higher initial rate of absorption than the hydrocolloids in the second hydrocolloid adhesive layer.

The hydrocolloid adhesive layer may have any thickness that is suitable for its intended use. Again, a comparatively light medical device requires a thinner hydrocolloid adhesive layer than a comparatively heavier medical device. By way of example, when the hydrocolloid adhesive layer is used to attach an ostomy plate, the preferred thicknesses for the hydrocolloid adhesive layer is 50 micron or more, more preferably 100 micron or more, more preferably 200 micron or more, more preferably 300 micron or more, more preferably 400 micron or more and more preferably 500 micron or more in view of providing sufficient adhesion for the ostomy plate and ostomy bag as it fills with bodily waste as well as providing sufficient moisture absorbing and retention properties. In terms of providing a more conformable and comfortable layer, the hydrocolloid adhesive layer preferably has a thickness of 2000 micron or less, more preferably 1500 micron or less, more preferably 1000 micron or less, more preferably 900 micron or less, more preferably 800 micron or less, and most preferably 700 micron or less. Any of the preferred lower limits for the hydrocolloid adhesive thickness can be combined with any of the preferred upper limits to define further suitable thickness ranges for the present invention. As an example, further exemplary ranges for the hydrocolloid adhesive thickness include: 50-2000 micron, 100-1500 micron, 200-1000 micron, 300-900 micron, 400-800 micron, 500-700 micron, 50-1000 micron, 100-1000 micron and 500-800 micron. The above values represent the thickness of the hydrocolloid adhesive layer before the adhesive system is used to attach a device to the skin. Where the thickness of the hydrocolloid adhesive layer varies across the adhesive system, then the above values refer to the average thickness of the hydrocolloid adhesive layer.

The hydrocolloid adhesive layer is preferably in the form of a continuous sheet, and preferably has the same shape and size as the underlying debondable adhesive layer. The hydrocolloid adhesive layer may also be interrupted by punched holes that influence moisture absorbency and any other gaps required to attach a medical device to the skin (e.g. having an aperture to allow access to the stoma of a patient).

### 5.3 Water permeable layer

The adhesive system of the present invention optionally comprises a water permeable layer interposed between the debondable adhesive layer and the hydrocolloid adhesive layer (e.g. see Figure 4). The water permeable layer can provide a partial or total physical separation of the debondable adhesive layer from the hydrocolloid adhesive layer. The presence of a water permeable layer provides further opportunity to tailor the adhesive system so that the hydrocolloid adhesive layer peels away from the debondable adhesive more readily and more cleanly than if the water permeable layer was not present. Furthermore, in the case than any leakage does occur, body effluent tends to gather between the water permeable layer and the hydrocolloid adhesive layer thus protecting the skin.

Water permeable layers are known in the art and any suitable layers can be employed in the adhesive system of the present invention such as film or sheet materials, woven or nonwovens (e.g. of polyester), textile substances or any of these materials rendered permeable by mechanical treatment (e.g. perforation). Examples of suitable materials include polyurethane, polyester, polyvinyl acetate, polyvinylidene chloride, polyethylene, polypropylene, polyethylene terephthalate, or the like, or composite materials thereof.

Thermoplastic films that provide a liquid barrier but provide water vapour permeability can also be used. Particularly preferred are hydrophilic continuous films that do not allow the flow of water vapour through open pores or apertures in the material, but do transfer substantial amounts of water vapour through the film by absorbing water on one side of the film where the moisture concentration is higher, and desorbing or evaporating it on the opposite side of the film where the water vapour concentration is lower. Such films are typically formed from a polymeric composition comprising a hydrophilic polymer, or a blend of hydrophilic polymers. Hydrophilic polymeric compositions having the above described characteristics are also known in the art as "monolithic compositions", and the water permeable, liquid impermeable layers or films made therefrom are known as "monolithic layers" or "monolithic films".

For example, US 5,447,783 describes a vapour permeable water resistant multi component film structure having at least three layers. The outer layers are hydrophobic copolyether-ester elastomers having a thickness of 1.3-7.6 micrometers and a Moisture Vapour Transmission Rate (MVTR) of 400-2500 g/m² · 24h and the inner layer is a hydrophilic copolyether-ester elastomer having a thickness of 7.6-152 micrometers and a MVTR of at least 3500 g/m² · 24h. US 4,493,870 describes a flexible layered waterproof product comprising a textile material covered with a film of a copolyetherester having a MVTR of at least 1000 g/m² · 24h having a thickness of 5 to 35 micrometers. GB 2024100 discloses a flexible layered water resistant article comprising a microporous hydrophobic outer layer which is moisture vapour permeable but resist liquids and a hydrophilic inner layer of polyetherpolyurethane having a MVTR of above 1000 g/m² · 24h.

Thermoplastic polymers that can be used for the water permeable layer according to the present invention may be selected from intrinsically breathable monolithic polymers and, according to a particular advantage of the present invention, also from non- or low breathable thermoplastic polymers. Suitable polymers for the water permeable layer include polyurethanes, poly-ether-amides, polyethylene-acrylic acid copolymers, polyethylene oxide and its copolymers, poly lactide and copolymers, polyamides, polyester block copolymers, sulfonated polyesters, poly-ether-esters, poly-ether-ester-amides, polyacrylates, polyacrylic acids and derivatives, ionomers, polyethylene-vinyl acetate, polyvinyl alcohol and its copolymers, polyvinyl ethers and their copolymers, styrene-maleic anhydride copolymers, poly-2-ethyl-oxazoline and derivatives, or mixtures thereof. Preferred polymers for the water permeable layer are polyethylene, polypropylene, and polyurethane.

The water permeable layer preferably has a MVTR of at least 700 g/m² · 24h, preferably at least 1,000 g/m² · 24h, more preferably at least 2,000 g/m² · 24h and most preferably at least 3,000 g/m² · 24h. The upper limit of the MVTR is not particularly limited but for practical purposes it may set at 15,000 g/m² · 24h or less, preferably 12,000 g/m² · 24h or less, more preferably 10,000 g/m² · 24h or less and most preferably 8,000 g/m² · 24h or less. Any of the disclosed MVTR lower limits can be combined with any of the disclosed MVTR upper limits to define a suitable MVTR range for the purposes of this invention. Exemplary MVTR ranges include 700-15,000 g/m² · 24h, 1,000-12,000 g/m² · 24h, 2,000-10,000 g/m² · 24h, 3,000-8,000 g/m² · 24h, 1,000-15,000 g/m² · 24h, and 1,000-10,000 g/m² · 24h. MVTR is measured by the Water Method according to ASTM E96-94 at 40°C and 20% relative humidity over a 24 hour period.

The water permeable layer may have any thickness that is suitable for its intended use. By way of example, when the hydrocolloid adhesive layer is used to attach an ostomy plate, the preferred thicknesses for the water permeable layer is 5 micron or more, more preferably 10 micron or more, more preferably 15 micron or more, more preferably 20 micron or more, more preferably 25 micron or more and more preferably 30 micron or more in view of providing sufficient barrier properties and support. In terms of providing a more conformable and comfortable layer, the water permeable layer preferably has a thickness of 200 micron or less, more preferably 150 micron or less, more preferably 100 micron or less, more preferably 90 micron or less, more preferably 80 micron or less, and most preferably 70 micron or less. Any of the preferred lower limits for the water permeable layer thickness can be combined with any of the preferred upper limits to define further suitable thickness ranges for the present invention. As an example, further exemplary ranges for the water permeable layer thickness include: 5-200 micron, 10-150 micron, 15-100 micron, 20-90 micron, 25-80 micron, 30-70 micron, 10-100 micron, 10-80 micron and 5-80 micron. Where the thickness of the water permeable layer varies across the adhesive system, then the above values refer to the average thickness of the water permeable layer.

The water permeable layer is preferably in the form of a continuous sheet, and preferably has the same shape and size as the underlying debondable adhesive layer and the overlying hydrocolloid adhesive layer. The water permeable layer may also be interrupted by punched holes that influence moisture absorbency and any other gaps required to attach a medical device to the skin (e.g. having a central aperture to allow access to the stoma of a patient) and/or to influence the adhesion level of the underlying debondable adhesive layer

### 5.4 Peel force

The debondable adhesive layer should have a peel force on skin (P_{D-S}) such that the medical device to be attached remains firmly attached until a debonding trigger can be applied. Naturally, the required peel force will depend on the intended use of the adhesive system where a comparatively heavy medical device requires that a debondable adhesive layer having a larger peel force be used than for a comparatively lighter medical device. Those skilled in the art can adjust the peel force of the debondable adhesive layer for the appropriate use, for example by a judicious choice of the type and amount of tackifier and/or plasticizer in the adhesive composition.

An advantage of the adhesive system according to the present invention is that the debondable adhesive layer can have a relatively high peel force on skin (e.g. 2-25 N/25mm) without the disadvantages that would typically result (e.g. skin trauma) when removing an adhesive layer with such a high peel force. The peel force of the debondable adhesive layer can be controllably reduced to a more acceptable and safe level before its removal by the application of a debonding trigger. The greater the debonding effect upon application of the trigger, the greater the initial peel force can be.

By way of example, if the adhesive system is used to attach an ostomy plate or bag, then P_{D-S} (that is, before a trigger is applied) is preferably at least 2 N/25mm, more preferably at least 4 N/25mm, even more preferably at least 5 N/25mm and most preferably at least 6 N/25mm. The upper limit for the peel force is not particularly limited as the debondable adhesive will be treated with a trigger so that it can easily be removed. For practical purposes, the upper limit of P_{D-S} can be set at 25 N/25mm or less, more preferably 22 N/25 m or less, even more preferably 20 N/25mm or less, and most preferably 18 N/25 mm or less. Any of the disclosed peel force lower limits for P_{D-S} can be combined with any of the disclosed peel force upper limits to define a suitable peel force range for P_{D-S} for the purposes of this invention. Exemplary peel force ranges include 2-25 N/25mm, 2-22 N/25mm, 4-20 N/25mm, 5-18 N/25mm, 5-20 N/25mm, 6-20 N/25mm and 4-18 N/25mm.

The peel forces recited above can be determined by Test Method A of the approved standard ASTM D3330M-04(2010) using the ear of a pig as the substrate. The ear should be clean so as to be free of debris or other agents that might affect the peel force measurement. For example, the ear can be cleaned in water to remove relatively large debris and then, after drying, pre-stripped with an acrylic-based adhesive (e.g. Transpore®, 3M, USA) to remove minor debris and any remaining agents that might affect the measurement. Obtaining a constant peel force (within experimental error) between two sequential pre-stripping applications can be used as an indication that the ear is sufficiently clean to determining the peel force in accordance with the invention. When measuring peel forces, the debondable adhesive layer should correspond to that (composition, thickness) which will be used in the adhesive system.

The hydrocolloid adhesive layer should have a peel force high enough such that the medical device to be attached remains firmly attached to the patient until it is time to remove the device. For instance, if the hydrocolloid adhesive layer is attached directly to the debondable adhesive layer, then the hydrocolloid layer should have a peel force on the debondable adhesive layer (P_{H-D}) such that the hydrocolloid layer remains firmly attached to the debondable adhesive layer until the hydrocolloid layer is removed. By way of example, if the adhesive system is used to attach an ostomy plate or bag, then P_{H-D} is preferably at least 1 N/25mm, more preferably at least 2 N/25mm, even more preferably at least 3 N/25mm and most preferably at least 4 N/25mm. The upper limit for the peel force should be such that the hydrocolloid adhesive layer can be removed from the debondable adhesive layer. For example, the upper limit of P_{H-D} can be set at 25 N/25mm or less, more preferably 22 N/25 m or less, even more preferably 20 N/25mm or less, and most preferably 18 N/25 mm or less. Any of the disclosed peel force lower limits for P_{H-D} can be combined with any of the disclosed upper limits to define a suitable peel force range for P_{H-D} for the purposes of this invention. Exemplary peel force ranges include 1-25 N/25mm, 2-22 N/25mm, 3-20 N/25mm, 3-18 N/25mm, 4-20 N/25mm, 1-20 N/25mm and 2-18 N/25mm.

Naturally, the peel force of the hydrocolloid layer on the medical device, or on any other substrate between it and the medical device, should also be high enough such that the medical device to be attached remains firmly attached to the patient until it is time to remove the device and/or adhesive system.

The peel forces recited above can also be determined by Test Method A of the approved standard ASTM D3330M-04(2010). In this case, the substrate to be used is the ear of a pig having a debondable adhesive layer adhered thereto. The debondable adhesive layer and the hydrocolloid adhesive layer should both correspond (composition, thickness) to those that will be used in the adhesive system in question.

In a preferred embodiment of the invention, the peel force P_{H-D} is less than the peel force P_{D-S}. This allows the hydrocolloid adhesive layer (and any medical device that is attached thereto) to be removed while leaving the debondable adhesive layer attached to the patient (e.g. see Figures 3a and 5a). A trigger can then be applied to the debondable adhesive layer to reduce its peel force (Figures 3b and 5b) and allow its removal more easily from the patient (Figures 3c and 5c). One advantage of first removing the hydrocolloid adhesive layer is that the trigger can be more easily applied to the debondable adhesive layer. This is particularly beneficial for light-triggered and temperature-triggered debondable adhesives as the hydrocolloid adhesive layer (and any medical device if still attached) tends to shield the adhesive from the light/temperature source.

The P_{H-D}/P_{D-S} ratio is preferably 0.95 or less, more preferably 0.90 or less, even more preferably 0.85 or less, and most preferably 0.80 or less in view of making it easier to preferentially remove the hydrocolloid adhesive layer. The P_{H-D}/P_{D-S} ratio is preferably 0.10 or more, more preferably 0.20 or more, even more preferably 0.30 or more and most preferably 0.40 or more in view of improving the adhesiveness of the hydrocolloid layer to the debondable adhesive layer. Any of the disclosed P_{H-D}/P_{D-S} ratio lower limits can be combined with any of the disclosed P_{H-D}/P_{D-S} ratio upper limits to define a suitable P_{H-D}/P_{D-S} ratio range for the purposes of this invention. Exemplary peel force ratios include 0.10-0.95, 0.20-0.90, 0.30-0.85, 0.40-0.80, 0.40-0.90, and 0.30-0.80. Preferably, the P_{H-D} and P_{D-S} values for determining the P_{H-D}/P_{D-S} ratio can be obtained by Test method A of ASTM D3330M-04(2010) as described previously. However, since the P_{H-D}/P_{D-S} value is a ratio, it can be obtained by any suitable method for determining peel forces provided that the same peel force measurement method is used to measure both P_{H-D} and P_{D-S}.

When the adhesive system comprises a water permeable layer interposed between the debondable adhesive layer and the hydrocolloid adhesive layer (e.g. Figure 4) then the relative peel force considerations discussed above can be adapted accordingly. In this case the peel force of the hydrocolloid adhesive layer on the water permeable layer (P_{H-W}) and the peel force of the water permeable layer on the debondable adhesive layer (P_{W-D}) can also be tailored. For instance, if P_{H-W} is greater than P_{W-D} then the water permeable layer can be removed from the debondable adhesive layer together with the hydrocolloid layer (e.g. Figure 5a). This will leave the debondable adhesive layer exposed so that a trigger can easily be applied. However, if P_{H-W} is less than P_{W-D} then the water permeable layer will remain attached to the debondable adhesive layer when the hydrocolloid adhesive layer is removed (e.g. Figure 6a). Leaving the water permeable layer in place has the advantage that a new hydrocolloid adhesive layer can be applied to the water permeable layer (4). This is most useful if a medical device needs to be temporarily removed (e.g. for calibration, cleaning, refilled, emptying) before being reattached. If needed, a subsequent hydrocolloid adhesive layer could be tailored such that P_{H-W} is now greater than P_{W-D} thereby facilitating the removal of the water permeable layer at a later time.

It will be appreciated that the ratio of P_{H-W}/P_{W-D} can be tailored according to need. If it is desired to remove the water permeable layer from the debondable layer when the hydrocolloid adhesive layer is removed then the P_{W-D}/P_{H-W} ratio is preferably 0.95 or less, more preferably 0.90 or less, even more preferably 0.85 or less, and most preferably 0.80 or less in view of making it easier to preferentially remove the water permeable layer. The P_{W-D}/P_{H-W} ratio is preferably 0.10 or more, more preferably 0.20 or more, even more preferably 0.30 or more and most preferably 0.40 or more in view of improving the adhesiveness of the water permeable layer to the hydrocolloid adhesive layer. Any of the disclosed P_{W-D}/P_{H-W} ratio lower limits can be combined with any of the disclosed P_{W-D}/P_{H-W} ratio upper limits to define a suitable P_{W-D}/P_{H-W} ratio range for the purposes of this invention. Exemplary peel force ratios include 0.10-0.95, 0.20-0.90, 0.30-0.85, 0.40-0.80, 0.40-0.90, and 0.30-0.80. If it is desired to leave the water permeable layer attached to the debondable layer when the hydrocolloid adhesive layer is removed, then the inverse of the ratios disclosed above are preferable. Or in other words, P_{H-W}/P_{W-D} can take any of the preferred upper, lower or ratio ranges disclosed immediately above. To allow the hydrocolloid adhesive layer be preferentially removed from the debondable adhesive layer (with or without the water permeable layer), P_{D-S} needs only be greater than P_{W-D} or P_{H-W}.

The peel forces recited above can also be determined by Test Method A of the approved standard ASTM D3330M-04(2010). In this case, the substrate to be used is the ear of a pig having a debondable adhesive layer adhered thereto. In the case of P_{H-W}, a water permeable layer is also adhered to the debondable adhesive layer. The debondable adhesive layer, the water permeable layer, and the hydrocolloid adhesive layer should all correspond (composition, thickness) to those that will be used in the adhesive system in question.

As before, it is preferable that the P_{H-W} and P_{W-D} values for determining the P_{W-D}/P_{H-W} or P_{H-W}/P_{W-D} ratios are obtained by Test method A of ASTM D3330M-04(2010). However, since the P_{W-D}/P_{H-W} or P_{H-W}/P_{W-D} value is a ratio, it can be obtained by any suitable method for determining peel forces provided that the same peel force measurement method is used to measure both P_{H-W} and P_{W-D}.

### 5.5 Manufacture & use

The adhesive layers can be prepared by coating a corresponding composition in its uncured form on a suitable substrate (e.g. a backing or a release liner) or on a medical device and then curing the composition using thermal energy or actinic radiation. For the coating purpose, any suitable coating techniques such as hand-proofing, slot-dye, extrusion coating, knife-coating, roll coating, knife-over-roll, blade coating or gravure coating can be used, depending upon the viscosity and other flow properties of the adhesive mixture.

One or more release liners can be used to cover the debondable adhesive layer and/or the hydrocolloid adhesive layer of the adhesive system during storage. If the adhesive system is attached to a medical device, then a release liner can be used to cover just the debondable adhesive of the adhesive system. In this case the release liner is removed from the adhesive system to expose the debondable adhesive which will be applied to the patient's skin. Typically the release liner has a very low peel force to allow its easy removal from an adhesive layer.

The release liner, if present, is not particularly limited, and examples of suitable materials are known in the field. Examples of such include plastic films of polyester-based resin film such as poly(ethylene terephthalate); vinyl-based resin film such as poly(vinyl chloride), poly(vinylidene chloride), polystyrene and the like; acrylic resin film such as various acrylic and methacrylic polymers; polycarbonate resin film; polyimide resin film; cellulose-based resin film such as acetyl cellulose, regenerated cellulose (cellophane), celluloid and the like; a laminate film of high-quality paper, glassine paper and the like and polyolefin-based film and the like. Commercially available release liners suitable for use in the present invention include MEDIRELEASE® 2500, MEDIRELEASE® 2249 and MEDIRELEASE® 2226, each manufactured by Mylan Technologies, Inc., Clearsil® release liner UV5A manufactured by CPFilms, Inc. or Scotchpak™ 1022, manufactured by 3M Pharmaceuticals/D.D.S. The thickness of the aforementioned release liner is generally 10 µm to 200 µm, preferably 25 µm to 100 µm.

The release liner is preferably treated for easy peeling on the interfacial surface side with an adhesive layer, so as to facilitate peeling from the adhesive layer. While the easy peeling treatment is not particularly limited, a known method can be applied. For example, a treatment method for forming a peeling-treated layer using a release agent containing a curable silicone resin as a main component by a coating method such as bar coating, gravure coating and the like can be mentioned. The thickness of the aforementioned peeling-treated layer is preferably 0.01 µm to 5 µm to ensure releasability and uniformity of the coating. The thickness of the release liner having a peeling-treated layer is generally 10 µm to 200 µm, preferably 50 µm to 100 µm, from the aspect of handling property.

The release liner in the adhesive system of the present invention may have an extended part outwardly extending over the adhesive layer. The extended part decreases the frequency of contact of the end of the adhesive layer against the inside of any packaging material that may be used to contain the adhesive system. The length of the aforementioned extended part of the release liner is preferably about 0.5 mm to 10 mm, more preferably about 1 mm to 3 mm, so as to achieve the aforementioned effects and ensure smooth insertion into a package.

The release liner may also have a back split part. The aforementioned back split part is made by forming a broken line on the surface on the opposite side from the contact surface of the release liner with the adhesive layer. The shape of the aforementioned broken line may be linear or curve (e.g., wave shape), or a combination of these. The broken line may be a solid line or a dashed line, or a combination of these. Since a release liner has a back split part, the release liner can be easily removed when the adhesive patch is used.

Suitable release liners include those known in the art for use with pressure sensitive adhesive composition. For example, the release liner can comprise a fluorosilicone coated polyester, silicone coated polyester or a UV cured, silicone-coated polyester. Preferred release liners include MEDIRELEASE® 2500, MEDIRELEASE® 2249 and MEDIRELEASE® 2226, each manufactured by Mylan Technologies, Inc., Clearsil® release liner UV5A manufactured by CPFilms, Inc. or Scotchpak™ 1022, manufactured by 3M Pharmaceuticals/D.D.S. The release liner, however, can comprise other materials, including paper or paper-containing layers or laminates, various thermoplastics, polyester films, foil liners, and the like.

The adhesive system of this invention can be used as a means of adhering an associated drug delivery system or a drug-containing delivery matrix to the skin for transdermal delivery of the drug. The adhesive system also can be used as a means for adhering a medical device such as a wound dressing, prosthesis, ostomy plate or bag, electrode, diagnostic device, insulin pumps catheter (e.g. central venous catheter or intravenous catheter), or active delivery device (e.g., iontophoresis, electrophoresis, electroporation, RF-ablation, micro-needles, phonophoresis, etc.) to skin for extended periods. The adhesive system is particularly suited for attaching ostomy plates and bags to the skin of a patient.

The adhesive system allows for the attachment of a medical device to a patient. In use the adhesive system is arranged such that the debondable adhesive layer faces towards the patient and the hydrocolloid adhesive faces away from the patient. The adhesive system can be applied to a patient and then the medical device secured to the adhesive system, or the adhesive system can be first applied to the medical device and then the medical device attached to the patient. In this case, the medical device can be supplied with the adhesive system already attached or it can be supplied separately to the adhesive system.

In use, moisture or bodily fluids that would otherwise affect the bonding properties of the debondable adhesive or cause skin irritation are drawn away through the water permeable layer (if present) and are absorbed by the hydrocolloid adhesive layer.

After the medical device has served its purpose, it can be removed from the patient, either together with the adhesive system or preferentially in multiple parts (e.g. first the medical device is removed from the adhesive system and then the adhesive system is removed from the patient; or the medical device is removed with part of the adhesive system as shown in Figures 3, 5 and 6; or the medical device and adhesive system are all removed together as shown in Figures 2). Advantageously, a trigger may be applied to the debondable adhesive so as to reduce the peel force required to remove the skin-adhering adhesive (with or without the medical device still attached) from the patient in a fast and clean way without causing any discomfort or trauma to the patient's skin. Furthermore, by allowing preferential removal of the medical device and/or hydrocolloid adhesive layer, the debondable adhesive layer becomes more accessible to any trigger that is to be applied. This is particularly beneficial for light debondable adhesives as the medical device tends to shield the debondable adhesive from the light source, as well as for temperature-triggered debondable adhesives as the device tends to shield heat transfer from the adhesive skin interface.

## Claims

1. An adhesive system for attaching a medical device to the skin of a patient, comprising:
a debondable adhesive layer; and
a hydrocolloid adhesive layer.

2. The adhesive system according to claim 1, wherein the debondable adhesive layer is in direct physical contact with the hydrocolloid adhesive layer.

3. The adhesive system according to claim 1 or claim 2, wherein the debondable adhesive layer has a peel force on skin (P_{D-S}) in the range of from 2 to 25 N/25mm as measured by Test Method A of ASTM D3330M-04(2010) on the ear of a pig.

4. The adhesive system according to any one of claims 1 to 3, wherein the debondable adhesive layer is in direct physical contact with the hydrocolloid adhesive layer, and the hydrocolloid layer has a peel force on the debondable adhesive layer (P_{H-D}) in the range of from 1 to 25 N/25mm as measured by Test Method A of ASTM D3330M-04(2010) using the ear of a pig with a debondable adhesive layer adhered thereto as the substrate.

5. The adhesive system according to any one of claims 1 to 4, wherein the debondable adhesive layer is in direct physical contact with the hydrocolloid adhesive layer, and the P_{H-D}/P_{D-S} ratio is less than 1, preferably in the range of from 0.10 to 0.95.

6. The adhesive system according to any one of claims 1 to 5, further comprising a water permeable layer interposed between the debondable adhesive layer and the hydrocolloid adhesive layer.

7. The adhesive system according to claim 6, wherein the water permeable layer separates the debondable adhesive layer from the hydrocolloid adhesive layer such that no direct physical contact is made between the planar surface of the two adhesive layers.

8. The adhesive system according to claim 6 or 7, wherein P_{D-S} is greater than the peel force of the hydrocolloid adhesive layer on the water permeable layer (P_{H-W}), or wherein P_{D-S} is greater than the peel force of the water permeable layer on the debondable adhesive layer (P_{W-D}), or wherein P_{D-S} is greater than the peel force of both P_{H-W} and P_{W-D}.

9. The adhesive system according to any one of claims 6 to 8, wherein the P_{W-D}/P_{H-W} ratio is in the range of from 0.10 to 0.95, or wherein the P_{H-W}/P_{W-D} ratio is in the range of from 0.10 to 0.95.

10. The adhesive system according to any one of claims 1 to 9, wherein the debondable adhesive layer is a light-triggered debondable adhesive or a temperature-triggered debondable adhesive.

11. The adhesive system according to any one of claims 1 to 10, wherein the debondable adhesive layer comprises a polymer selected from acrylics, rubbers, silicones, polyurethanes, polyesters, polyethers, ethylene-vinyl acetate, polyacrylate or styrene/butadiene copolymers, or copolymers, grafts or mixtures thereof.

12. The adhesive system according to any one of claims 1 to 11, wherein the hydrocolloid adhesive layer comprises a polymer selected from cellulosics, such as carboxymethyl cellulose (CMC), chitosans, pectins, guar gums, starches, dextrines, collagens, gelatines, polyacrylic acids, polyvinylealcohol/acetates, polyhydroxyalkyl acrylates, polyhydroxyalkyl methacrylates, polyacrylamides, polystyrene sulfonates, polyvinylpyrrolidones, polyglycols, or copolymers, grafts or mixtures thereof.

13. The adhesive system according to any one of claims 1 to 12, wherein the water permeable layer comprises a polymer selected from polyurethane, polyester, polyvinyl acetate, polyvinylidene chloride, polyethylene, polypropylene, and polyethylene terephthalate,

14. A medical device comprising the adhesive system according to any one of claims 1 to 13

15. The medical device according to claim 14, which is a wound dressing, a prosthesis, an ostomy faceplate or bag, an electrode, a diagnostic device, an insulin pump, or a catheter.
